# EUROPEAN PATENT APPLICATION

(11) **EP 0 556 745 A1**
(43) Date of publication of application: **25.08.1993**
(21) Application number: 93102241.2
(22) Date of filing: 12.02.1993
(51) Int. Cl.: G01N 33/68, G01N 33/543

(54) **Method for detection of anti-wheat-protein antibodies**

(30) Priority: 21.02.1992 US 839611
(71) Applicant: ABBOTT LABORATORIES, Abbott Park, Illinois 60064-3500 (US)
(72) Inventor: Grier, Thomas J., Grayslake, Illinois 60030 (US); Anawis, Mark A., Grayslake, Illinois 60030 (US)
(74) Representative: Modiano, Guido, Dr.-Ing.

(57) **Abstract**

A method for the detection of anti-wheat-protein IgE antibodies in patient body fluids. A composition of wheat protein extract is useful for the detection of anti-wheat-protein IgE antibodies. The method involves applying to a solid phase an amount of the composition in solution and drying. The solid phase is placed in contact with a patient test sample and the presence or amount of anti-wheat-protein IgE antibodies is determined.

## Description

This is a continuation-in-part application of co-pending U.S. Patent Application Serial No. 07/794,812 filed November 15, 1991, entitled "BINDING OF MILK ALLERGENS TO A SOLID PHASE", which is a continuation-in-part application of co-pending U.S. Patent Application Serial No. 07/509,255 filed April 13, 1990, entitled "BINDING OF ALLERGENS TO A SOLID PHASE", both of which enjoy common ownership and are incorporated herein by reference. This application is also a related application to U.S. Patent Application Serial No. 07/227,272 filed August 2, 1988, entitled "TEST CARD FOR PERFORMING ASSAYS", which also enjoys common ownership and is incorporated herein by reference.

### BACKGROUND OF THE INVENTION

The present invention relates to the binding of antigens related to wheat allergens to a solid phase material for use in a diagnostic assay for immunoglobulins that bind to said allergens. In particular, the invention improves the sensitivity of a diagnostic assay for immunoglobulins, suspected of being present in a blood sample, that bind to proteins found in wheat.

U.S. Patent No. 3,720,760 discloses that certain immunogenic substances, called allergens, can give rise to allergic reactions in the form of asthma, hay fever, and the like, and that the blood of a patient in whom a given allergen causes an allergic reaction usually contains low concentrations of immunoglobulins, called reagin-immunoglobulins (now usually called "IgE", which term is subsequently used herein), which are directed specifically against that allergen. The patent discloses a test for sensitivity to allergens which involves injecting given allergens into the skin of a patient; a skilled observer then assesses the degree of sensitivity to each of the allergens on the basis of the observed reaction (reddening or swelling of the skin) caused by each allergen. The patent also discloses an "in vivo" test, where the patient inhales an allergen in the form of an aerosol, and the patient is deemed to be sensitive to any allergen that causes hay fever, asthma or like symptoms.

The patent further discloses an in vitro method for determining the presence of IgE antibodies in a body fluid. The method involves binding an allergen to fine particles of a copolymer, e.g., a dextran-epichlorohydrin copolymer, by treating the particles with cyanogen bromide, and suspending the particles and an allergen in an aqueous medium. A body fluid to be tested for the presence of IgE antibodies directed against that allergen is then contacted with the allergen bound to the copolymer. The product of step (2) is then brought into contact with radio-labeled antibodies which will bind to IgE antibodies, if any, that have become bound to the allergen that is bound to the copolymer. The radiation emitted from the solids of step (3), the liquid of step (3), or both can then be measured.

The covalent coupling of antigens, including allergens, to a solid phase was used to prevent or inhibit their removal from the solid phase during the assay procedure. U.S. Patent No. 4,597,999 describes the covalent coupling of two molecular species to one another, using cross-linking agents having at least two functional groups which are subject to independent activation. Examples of such cross-linking agents include 4-methylazidobenzimidate and N-hydroxysuccinimidylazidobenzoate. These cross-linking agents couple spontaneously in the dark to available amino groups, as in aminopropyl glass, aminophenyl glass and aminohexylagarose, and when activated by irradiation with light of a suitable wavelength, these agents also couple with a ligand such as a drug, digoxin, a steroid, or a protein.

U.S. Patent No. 4,425,434 describes the use of biologically active substances to fill the pores of porous titania spheroids, porous calcium phosphate spheroids, porous zirconia spheroids, or similar porous support material, and that the biologically active substance can then be immobilized in the pores by precipitation and cross-linking. The biologically active substance can be a proteinaceous substance, such as an enzyme.

It has been found, however, that the covalent coupling procedures are costly to perform and time consuming. In addition, some coupling procedures can decrease the sensitivity of the assay.

Wheat contains a variety of proteins including albumins, globulins, gliadins and glutenins. Wheat allergy is defined as those reactions induced by the ingestion of wheat or its components for which an immunological pathogenesis may be demonstrated.

Pharmacia Diagnostics (Piscataway, New Jersey) markets in vitro radioimmunoassay and enzyme immunoassay allergy products (Phadebas RAST® and Phadezym® RAST) that detect and quantitate IgE antibodies for a variety of allergens including wheat and wheat proteins. The Pharmacia tests are performed individually utilizing a disk having the allergen of interest bound thereto. The disk is incubated with the patient sample, washed, incubated with labeled goat anti-human-IgE IgG and washed and then the amount of label on the disk is measured. Pharmacia markets tests for wheat and gluten.

### BRIEF DESCRIPTION OF THE PRESENT INVENTION

The present invention involves novel allergen compositions, using a solvent such as deionized or distilled water, containing (in milligrams of protein per milliliter, as determined by a suitable protein test): from about 0.05 to about 4.0 of *Alternaria alternata* allergen; from about 0.5 to about 50 *Aspergillus fumigatus* allergen; from about 0.8 to about 81.6 of Bermuda grass (*Cynodon dactylon*) allergen; from about 0.1 to about 6.0 of birch (*Betula nigra*) allergen; from about 0.6 to about 20.6 of cat (*Felis domesticus*) allergen; from about 0.04 to about 4.5 of mountain cedar (*Juniperus ashei*) allergen; from about 0.1 to about 20.5 of Japanese cedar (*Cryptomeria japonica*) allergen; from about 0.05 to about 10.0 of *Cladosporium* allergen; from about 1.3 to about 38.4 of dog (*Canis familiarus*) allergen; from about 0.7 to about 22.4 of *Dermatophagoides farinae* (*D. farinae*) allergen; from about 0.6 to about 84.2 of *D. pteronyssinus* allergen; from about 0.1 to about 10.0 of elm (*Ulmus*) allergen; from about 0.02 to about 2.0 of feather allergen; from about 0.2 to about 20.5 of giant ragweed (*Ambrosia trifida*) allergen; from about 0.4 to about 100 of house dust allergen; from about 0.05 to about 10.5 of June/Kentucky bluegrass (*Poa pratensis*) allergen; from about 0.2 to about 20.5 of lamb's quarters (*Chenopodium album*) allergen; from about 0.1 to about 11.5 of maple (*Acer*) allergen; from about 0.3 to about 90.4 of mugwort (*Artemesia heterophylla*) allergen; from about 0.1 to about 12 of mulberry (*Morus*) allergen; from about 0.2 to about 25.5 of oak (*Quercus*) allergen; from about 0.1 to about 66.8 of olive (*Olea europea*) allergen; from about 1.0 to about 40.0 of *Parietaria* (*Parietaria officinalis*) allergen; from about 1.7 to about 130.4 of plantain (*Plantago lanceolata*) allergen; from about 0.1 to about 4.8 of *Penicillium* (*Penicillium notatum*) allergen; from about 0.05 to about 8.5 of perennial rye (*Lolium perenne*) allergen; from about 0.2 to about 20.5 of short ragweed (*Ambrosia elatior*) allergen; from about 0.05 to about 6.6 of timothy (*Phleum pratense*) allergen; from about 0.5 to about 50 mg/ml of milk protein extract supplemented with other proteins; and from about 0.3 to about 50 mg/ml of wheat allergen extract as disclosed in the present invention. Such allergen concentrations have been found optimal for the preparation of immunoassay devices for the detection of IgE antibodies specific for the allergens.

The present invention also involves devices for detecting the presence or amount of IgE antibodies in a test sample. The assay devices include a solid phase and an allergen immobilized upon the solid phase, wherein the allergen is typically applied as one of the above allergen compositions. In certain assay devices, the allergen composition is combined with a pretreatment substance such as a denaturant, organic solvent, cross-linking agent or concentrated salt solution. It has been unexpectedly found that such allergen pretreatment can enhance allergen immobilization upon the solid phase. The reaction or binding area of the solid phase can be optionally modified by the addition of a protein blocking reagent. Suitable blocking reagents include equine serum albumin, bovine serum albumin, fish gelatin, casein and the like.

In addition, the present invention describes allergen compositions containing a solvent and an allergen solubilized in the solvent (thereby forming an allergen solution), and a pretreatment substance chosen from denaturants, organic solvents, cross-linking agents or concentrated salt solutions, wherein the allergen solution is combined with the pretreatment substance, and wherein the resultant composition is used for the in vitro detection of the presence or amount of IgE antibodies in a test sample. In particular, the present invention describes a composition of wheat proteins which enhance the sensitivity of the in vitro detection of the presence or amount of anti-wheat-protein IgE antibodies. In vitro detection methods can involve: providing a solid phase prepared by applying the novel allergen compositions or pretreated allergen compositions to the solid phase; contacting the sample to be tested to that solid phase, thereby immobilizing allergen-specific IgE antibody from the sample upon the solid phase by forming allergen/antibody complexes; and detecting that immobilized allergen-specific antibody to determine the presence or amount of the antibody in the test sample. Generally, the solid phase is contacted with an indicator reagent to determine the presence or amount of IgE antibodies in the test sample, wherein the indicator reagent includes a label conjugated to a binding member that is specific for either the allergen, IgE antibodies or an ancillary specific binding member. The label that is selected is not critical to the present invention and is typically chosen from chromogens, catalysts, fluorescent compounds, chemiluminescent compounds, radioactive isotopes, colloidal metallic particles, colloidal selenium particles, dye particles, enzymes, substrates, organic polymer latex particles and liposomes or other vesicles containing signal producing components. The present invention also includes assay kits containing the allergen or allergens of interest immobilized upon the solid phase and a suitable indicator reagent. Optionally, the kit can include assay buffers and wash reagents.

### BRIEF DESCRIPTION OF THE DRAWINGS

FIG. 1 is an enlarged top plan view of a preferred embodiment of a reaction cartridge support having a well containing a test card which has discrete test sites with moats.

FIG. 2 is a magnified view, partially cutaway, through lines 8-8 (FIG. 1) showing sample test sites and moats in a preferred laminate structure of the test card.

FIG. 3 is a plot of anti-milk-protein IgE antibody containing plasma sample pools analyzed on a solid phase containing only milk protein extract and on a solid phase containing the milk protein composition of milk protein extract, casein and β-lactoglobulin. The relative color intensities (with background subtracted) of the solid phase test sites (measured by reflectance) are plotted against the concentration of IgE antibodies (PRU/ml) in the pools.

FIG. 4 is a plot of anti-wheat-protein IgE antibody containing plasma sample pools analyzed on a solid phase containing unmodified wheat allergen extract and on a solid phase containing heat modified wheat allergen extract. The relative color intensities with background subtracted (ΔDr) of the solid phase test sites (measured by reflectance) are plotted against the concentration of IgE antibodies (PRU/ml) in the pools.

### DESCRIPTION OF THE PREFERRED EMBODIMENTS

The present invention is based upon the discovery that an allergen solution can be used to bind an allergen to a solid phase material without the need for covalent linkages. A solid phase so prepared can then be used in an in vitro diagnostic assay for IgE antibodies. Suitable solid phase materials include cellulose nitrate or a mixed ester cellulose. In addition, it has been discovered that certain allergen concentrations are optimum insofar as the sensitivity of the assay is concerned. In particular, it has been discovered that a composition containing certain wheat protein concentrations are optimum insofar as the sensitivity of an assay for anti-wheat-protein IgE antibodies is concerned.

The invention is also based upon the discovery that many allergens can be pretreated to improve their adherence to the solid phase material. The allergen pretreatment methods of the present invention serve to enhance the binding of the allergen to the solid phase throughout the assay. The allergen pretreatment compositions and methods were also unexpectedly found to increase the amount of allergen which can be bound to the solid phase thereby enabling the binding of allergen in an amount that is optimal for the assay.

The present invention involves novel allergen compositions for the preparation of solid phase devices used in binding assays. The allergen compositions have been unexpectedly found to enhance the binding of the allergen to the solid phase material. As a result, greater amounts of antigen may be immobilized upon the solid phase, thereby providing more antigenic sites for binding antibody during the assay.

The present invention also involves the pretreatment of certain allergen compositions with substances such as denaturants, organic solvents, cross-linking agents, and concentrated salt solutions. Pretreatment of an allergen composition with one or more of these substances was unexpectedly found to enhance the adherence of the allergen to a solid phase throughout the assay procedure which may include multiple washing steps or other manipulations which could otherwise dislodge the allergen form the solid phase. In addition, the pretreatment of the allergen improves their binding performance at elevated temperatures often used in binding assays.

The preferable pretreatment of wheat involves dissolving the wheat allergen extract in a buffer and heating the solution. More preferably, the buffer is HEPPS buffer at a concentration within the range of about 0.01M to about 10M at a pH within the range of about 7.0 to about 9.0 and heating the solution at a temperature within the range of about 50°C to about 100°C for about 1 minute to about 20 minutes. Most preferably, the buffer is HEPPS buffer at a concentration of about 0.2M at pH of about 8.3 and the solution is heated at a temperature of about 80°C for three minutes.

Suitable denaturants include, but are not limited to: acids such as hydrochloric acid (HCl) and acetic acid. Organic solvents, such as tetrahydrofuran, are suitable for allergen pretreatment. Concentrated salt solutions, such as concentrated solutions of sodium chloride (NaCl), are also suitable for allergen pretreatment according to the present invention. Suitable cross-linking agents for the pretreatment of allergens include, but are not limited to: formaldehyde, glutaraldehyde and 1-ethyl-3-(3-dimethylaminopropyl)carbodiimide (EDAC).

Allergen compositions combined with such pretreatment substances are then used in the production of novel solid phase assay devices. The allergen compositions or pretreated allergen compositions are applied to a solid phase material upon which the allergen composition is dried and thereby immobilized. The solid phase devices can then be used in binding assays which include, but are not limited to, competitive assays, sandwich assays and indirect assays, and include both forward and reverse assay formats.

In a preferred embodiment of the present invention, the allergen of interest is immobilized upon a solid phase material made of nitrocellulose or a nitrocellulose derivative or compound, such as cellulose acetate/nitrate mixed ester cellulose. The maximum binding capacity of nitrocellulose for the protein bovine serum albumin is about 140 µg/cm². This binding capacity value is converted according to the desired size of the solid phase reaction or binding area of the present invention, and a value of 2.2 mg/ml is obtained. This concentration is used as the starting protein concentration for all allergens, but the optimum allergen concentration may be above or below this value. Different concentrations of allergen solutions are pretreated, immobilized on nitrocellulose and tested with a positive test sample, as described in the specific examples which follow. The allergen concentration is adjusted such that when concentration is plotted against signal a parabolic curve is obtained, and the optimum allergen concentration can be determined from the maximum detected signal.

The allergen protein concentrations which were tested ranged from about 0.05 milligrams of allergen per milliliter of solvent, prior to pretreatment, to about 170 mg/mL. The most effective concentration ranges for each of the allergens tested are presented in the specific examples which follow.

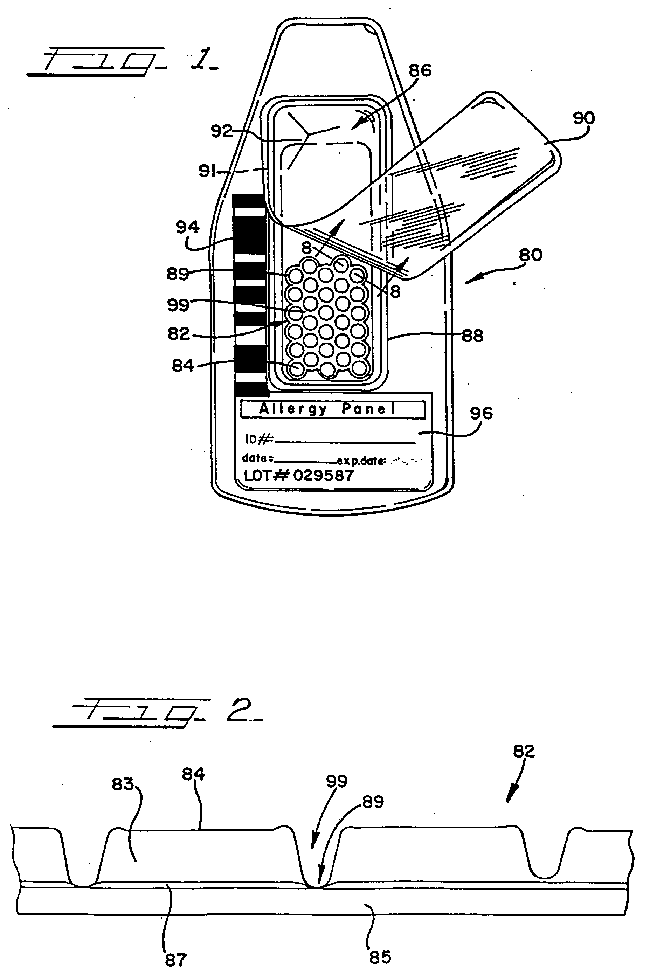

A preferred solid phase is illustrated in Figures 1 and 2. A preferred support for the solid phase is test card 82, as illustrated in FIG 1. Test card 82 preferably contains circular depressions 89 in the binding layer material which create an array of isolated test sites 84 preferably in close proximity to each other and each composed of binding layer material encircled by a moat 99 of air space. Test card 82 is preferably adhered to reaction cartridge 80 using two-sided adhesive tape on the bottom of reaction well 86, which is defined by well wall 88. Reaction well 86 is preferably provided with a removable, preferably transparent well cover 90 which preferably includes a reagent port 92 to facilitate the delivery and removal of fluids from the reaction well 86. The reaction cartridge 80 also preferably includes code means 94, such as an optical bar code, adapted to be read by an optical reader (not shown) and which is attached to or printed directly on the flat surface 91. The reaction cartridge 80 also preferably includes a panel 96 which may include information such as the expiration date of the particular reaction cartridge, the lot number of the particular panel of capture reagents or assay binding components and the like.

FIG. 2 is a magnified view, partially cutaway, through lines 8-8 of FIG. 1 showing sample test sites and moats in a preferred laminate structure of the test card. The test card is preferably a laminate structure comprising a binding layer 83 adhered to a non-absorbent substrate 85 using an adhesive 87. The porous structure of nitrocellulose has been found to have excellent absorption and adsorption qualities for a wide variety of fluid capture reagents which may be used in connection with the invention and is therefore preferred for binding layer 83. Polyester film such as MYLAR plastic having a thickness of approximately 0.002 inches is suitable for non-absorbent substrate 85. An adhesive backed polyester film is commercially available from several sources, such as Flexcon (Spencer, MA). A laminate structure suitable for use in test cards is commercially available from Millipore (Bedford, MA).

A different analyte or allergen is delivered to each test site 84 so that a single sample can be simultaneously tested for the presence of binding components specific to each of a panel of different capture reagents. Some test sites 84 may have analyte delivered thereto to serve as positive control sites and some may have no reagent delivered thereto, to serve as negative control or reference sites. Preferably, from about 1.1 to about 5 µL and more preferably, from about 1.25 to about 4 µL of analyte or allergen solution is delivered to each test site 84 using any number of suitable delivery methods including reagent jetting, metered air pulsing, positive displacement pump, or by capillary tube lowered to the surface of the test site 84.

After the test sites 84 of a test card 82 are spotted with analyte or allergen, the test sites are allowed to dry thoroughly at room temperature. After drying is complete, the binding layer 83 of test card 82 is preferably "blocked" with a protein coating such as inactivated horse serum or fish gelatin. Blocking masks potential non-specific binding sites on the binding layer 83. Suitable blocking is obtained during an incubation period of about 1 hour at approximately 37°C and is preferably accomplished in tanks with agitation during incubation. Following blocking, the test card 82 is washed, such as with 10 mM Tris buffered saline, and allowed to dry overnight.

The invention will be more fully understood from the following examples, which constitute the best modes presently contemplated by the inventors. It is to be understood, however, that the examples are presented solely for the purpose of illustration, and are not to be construed as limiting.

Before proceeding with the description of the specific embodiments of the present invention, a number of terms will be defined. All allergen contents herein refer to the protein content of the allergen solutions, determined using a suitable protein test such as Coomasie blue or Ninhydrin as are well-known in the art.

The term "analyte" refers to the substance to be detected in or separated from test sample. The analyte can be any substance for which there exists a naturally occurring specific binding member or for which a specific binding member can be prepared. In addition, the analyte may bind to more than one specific binding member. "Analyte" also includes any antigenic substances, haptens, antibodies, and combinations thereof. In the present invention, the main analytes to be detected or measured are IgE antibodies.

The term "test sample" refers to virtually any liquid sample. The test sample can be derived from any desired source, such as a physiological fluid, for example, blood, saliva, ocular lens fluid, cerebral spinal fluid, sweat, urine, milk, ascites fluid, mucous, synovial fluid, peritoneal fluid, amniotic fluid or the like. The liquid test sample can be pretreated prior to use, such as preparing plasma from blood, diluting viscous liquids, or the like; methods of treatment can also involve separation, filtration, distillation, concentration, inactivation of interfering components, and the addition of reagents. In addition, a solid can be used once it is modified to form a liquid medium.

The term "specific binding member" refers to a member of a specific binding pair, i.e., two different molecules wherein one of the molecules through chemical or physical means specifically binds to the second molecule. In addition to antigen and antibody specific binding pairs such as the allergen and antibody pair, other specific binding pairs include, biotin and avidin, carbohydrates and lectins, complementary nucleotide sequences, complementary peptide sequences, effector and receptor molecules, enzyme cofactors and enzymes, enzyme inhibitors and enzymes, a peptide sequence and an antibody specific for the sequence protein, polymeric acids and bases, dyes and protein binders, peptides and specific protein binders (e.g., ribonuclease, S-peptide and ribonuclease S-protein), and the like. Furthermore, specific binding pairs can include members that are analogs of the original specific binding member, for example an analyte-analog. If the specific binding member is an immunoreactant it can be, for example, an antibody, antigen, hapten, or complex thereof. If an antibody is used, it can be a monoclonal or polyclonal antibody, a recombinant protein or antibody, a mixture or mixtures or a fragment or fragments thereof, as well as a mixture of an antibody and other specific binding members. The details of the preparation of such antibodies and their suitability for use as specific binding members are well-known to those skilled-in-the-art.

An "indicator reagent", as used herein, refers to a label attached to a specific binding member. The indicator reagent produces a detectable signal at a level relative to the amount of an analyte in the test sample. Generally, the indicator reagent is detected or measured after it is captured on the solid phase material, but the unbound indicator reagent can also be measured to determine the result of an assay. The specific binding member component of the indicator reagent enables the indirect binding of the label to the analyte, to an ancillary specific binding member, to the capture reagent or to a complex thereof.

The term "label" refers to any substance which is attached to a specific binding member and which is capable of producing a signal that is detectable by visual or instrumental means. Suitable labels for use in the present invention can include chromogens; catalysts; fluorescent compounds; chemiluminescent compounds; radioactive isotopes; direct visual labels including colloidal metallic and non-metallic particles, dye particles, enzymes or substrates, or organic polymer latex particles; liposomes or other vesicles containing signal producing substances; and the like.

Many enzymes suitable for use as labels are disclosed in U.S. Patent No. 4,275,149, columns 19-23, herein incorporated by reference. For example, an enzyme/substrate signal producing system useful in the present invention is the enzyme alkaline phosphatase wherein the substrate used can be 5-bromo-4-chloro-3-indolyl phosphate or a derivative or analog thereof. If horseradish peroxidase is used, o-phenylenediamine or 4-chloro-naphthol is added as an enzyme substrate to form a colored product which can be detected and/or measured visually or instrumentally.

In an alternative signal producing system, the label can be a fluorescent compound where no enzymatic manipulation of the label is required to produce a detectable signal. Fluorescent molecules such as fluorescein, phycobiliprotein, rhodamine and their derivatives and analogs are suitable for use as labels in this system.

An especially preferred class of labels includes the visually detectable, colored particles which enable a direct colored readout of the presence or concentration of the analyte in the test sample without the need for using additional signal producing reagents. Materials for use as such particles include colloidal metals, such as gold, and dye particles as disclosed in U.S. Patent Numbers 4,313,734 and 4,373,932. The preparation and use of non-metallic colloids, such as colloidal selenium particles, are disclosed in co-owned and copending U.S. Patent Application Serial No. 072,084, filed July 9, 1987, which is incorporated by reference herein in its entirety. Organic polymer latex particles for use as labels are disclosed in co-owned and copending U.S. Patent Application Serial No. 248,858, filed September 23, 1988, which is incorporated by reference herein in its entirety.

A variety of different indicator reagents can be formed by varying either the label or the specific binding member; it will be appreciated by one skilled-in-the-art that the choice involves consideration of the analyte to be detected and the desired means of detection. The selection of a particular label is not critical, so long as the label is capable of generating a detectable signal either by itself or in conjunction with one or more additional signal producing components. The details of the preparation of such label/specific binding member conjugates are well-known to those skilled-in-the-art.

The term "signal producing component" refers to any substance capable of reacting with another assay reagent or the analyte to produce a reaction product or signal that indicates the presence of the analyte and that is detectable by visual or instrumental means. "Signal production system", as used herein, refers to the group of assay reagents that are needed to produce the desired reaction product or signal. For example, one or more signal producing components can be used to react with a label and generate the detectable signal, i.e., when the label is an enzyme, amplification of the detectable signal is obtained by reacting the enzyme with one or more substrates or additional enzymes to produce a detectable reaction product.

The term "capture reagent" refers to a capture binding member which is attached to a solid phase material to enable the separation of the analyte or indicator reagent, that is bound thereto, from unbound analyte and assay reagents. Typically, the attachment of the capture binding member to the solid phase material is substantially irreversible.

In forming a capture reagent to be used in an assay, once the capture binding member, e.g., allergen, is immobilized upon the solid phase, the remaining surface area of the solid phase is generally blocked with a suitable inactivating solution, such as bovine or equine serum albumin, casein or other proteinaceous material, to prevent non-specific binding of protein to the solid phase when the reaction mixture containing a specific binding member is contacted to the solid phase. The solid phase is then washed with an appropriate solution to remove any excess blocking solution and/or unbound capture binding member.

Once complex formation occurs between the assay components, the solid phase can be used as a separation mechanism. For example, the reaction mixture can be contacted to the capture reagent, and the solid phase material retains the newly formed reaction complex(es).

Assay devices can have many configurations, several of which are dependent upon the material chosen for the solid phase. The term "solid phase material" refers to any suitable chromatographic, bibulous, porous or capillary material or other conventional solid material, well-known to those skilled-in-the-art for use in immobilizing specific binding members. Solid phase materials can include fiberglass, nylon or cellulose or derivatives thereof, such as cellulose nitrate or a cellulose acetate/cellulose nitrate mixed ester cellulose. The solid phase, however, is not limited to porous materials. The solid phase material can also include, without limitation, polymeric or glass beads, microparticles, tubes, sheets, plates, slides, magnetic beads, a microtiter plate with one or more reaction wells or a glass or plastic test tube, or the like.

Natural, synthetic or naturally occurring materials that are synthetically modified, can be used as a solid phase material including polysaccharides, e.g., cellulose materials including paper, cellulose and cellulose derivatives such as cellulose acetate, nitrocellulose and cellulose acetate/nitrate mixed ester cellulose; silica; fiberglass; inorganic materials such as deactivated alumina, diatomaceous earth or other inorganic finely divided material uniformly dispersed in a porous polymer matrix, with polymers such as vinyl chloride, vinyl chloride-propylene copolymer, and vinyl chloride-vinyl acetate copolymer; cloth, both naturally occurring (e.g., cotton) and synthetic (e.g., nylon); porous gels such as silica gel, agarose, dextran and gelatin; polymeric films such as polyacrylamide; magnetic particles; microtiter plates; polystyrene tubes; protein binding membranes; agarose; Sephadex® (Pharmacia Fine Chemicals, Inc., Piscataway, New Jersey); Trisacryl (Pointet-Girard, France); silicon particles; porous fibrous matrixes; and the like. The solid phase material should have a reasonable inherent strength or strength can be provided by means of a support, and it should not interfere with the production of a detectable signal.

Optionally, the specific binding member of the capture reagent can be affixed to particles, e.g., microparticles. These microparticles can serve as the solid phase material and be retained in a column, suspended in a mixture of soluble reagents and test sample, or retained and immobilized by another solid phase base material. By "retained and immobilized" is meant that the microparticles, associated with the solid phase base material, are not capable of substantial movement to positions elsewhere within that material. The microparticles can be selected by one skilled-in-the-art from any suitable type of particulate material including those composed of polystyrene, polymethylacrylate, polypropylene, polytetrafluoroethylene, polyacrylonitrile, polycarbonate or similar materials. The size of the microparticles is not critical, although it is preferred that the average diameter be smaller than the average pore size of the solid phase base material if such is used.

The term "ancillary specific binding member" refers to a specific binding member used in addition to the specific binding members of the capture reagent and the indicator reagent. One or more ancillary specific binding members can be used in an assay. For example, an ancillary specific binding member can be used in an assay where the specific binding member of the indicator reagent is capable of binding the ancillary specific binding member which is in turn capable of binding the analyte.

The present invention is concerned with immunoassays. Therefore, the following discussion of immunoassays and definitions of terms often used with respect to immunoassays are set forth to facilitate the understanding of the disclosure and claims hereof.

In accordance with one method of the present invention, a sandwich assay can be performed wherein a capture reagent can include an allergen which has been bound to a solid phase material. The capture reagent is contacted with a test sample, suspected of containing the analyte, and an indicator reagent containing an analyte-specific binding member conjugated to a label. The reagents can be contacted to the sample simultaneously or added sequentially. A binding reaction results in the formation of a capture reagent/analyte/indicator reagent complex. The assay may also involve a washing step to separate the resultant complex from the excess reagents and test sample. Either the unreacted indicator reagent or the complex retained upon the solid phase is then observed to detect or measure the amount of label associated therewith. If analyte is present in the sample, then label will be present on the solid phase material. The amount of label on the solid phase is proportional to the amount of analyte in the sample.

The present invention also can be used to conduct a competitive assay. In one example of a competitive configuration, the capture reagent again includes a specific binding member (allergen) which has been attached to a solid phase material. The capture reagent is contacted with both test sample and an indicator reagent that includes an analyte or analyte analog which has been labeled with a signal generating compound. The indicator reagent and analyte then compete in binding to the capture reagent. The competitive binding reaction results in the formation of capture reagent/analyte complexes or capture reagent/indicator reagent complexes. The capture reagent/indicator reagent complexes can be detected via the label of the indicator reagent. In the competitive assay, the amount of label that becomes associated with the solid phase is inversely proportional to the amount of analyte in the sample.

The present invention can also be used in indirect immunoassays involving one or more ancillary specific binding members. For example, an indirect sandwich immunoassay with the formation of a capture reagent/analyte/anti-analyte antibody/indicator reagent complex can be performed, wherein the indicator reagent is a specific binding partner for the ancillary specific binding member which is specific for the analyte. The present invention can also be used in forward and reverse immunoassay protocols

The following examples are illustrative of the invention and are in no way to be interpreted as limiting the scope of the invention, as defined in the claims. It will be appreciated that one skilled in the art can conceive of many other devices and methods of use to which the present inventive concepts can be applied.

### Example 1

In this experiment, *Alternaria alternata* allergen was pretreated for binding to a solid phase material. A 37% aqueous formaldehyde solution (12.5 µL) was mixed with 100 microliters of a solution of *Alternaria alternata* (28.8 µg/mL) in deionized water. The amount of formaldehyde effective for pretreatment was found to range from about 10 µL to about 20 µL when the 37% aqueous formaldehyde solution was used. The resulting mixture was incubated at 4°C for about 10 hours, and the incubated composition was allowed to stand for 30 to 60 minutes at about 20°C. The mixture was then centrifuged, and the resultant supernatant, a pretreated *Alternaria alternata* allergen composition, was decanted. The pretreated composition was poured onto a disc of microporous cellulose nitrate (about 140 µm thick and about 3 mm in diameter) and allowed to dry. The allergen was thereby immobilized upon the solid phase material. The remaining surface of the disc was then blocked with a ten percent horse serum solution.

The solid phase bound allergen, or *Alternaria alternata* capture reagent, was then used in an enzyme immunoassay ("EIA"). The EIA method included the following steps. The sample to be tested (e.g., serum) was contacted to the capture reagent, thereby immobilizing allergen-specific IgE antibodies upon the solid phase. Optionally, the antibody immobilization step was followed by a wash step to remove unbound sample. The capture reagent was then contacted to an enzyme-labeled anti-IgE antibody (indicator reagent) which bound to that IgE antibodies from the sample, if any, which had bound to the solid phase. The solid phase was then washed to remove unbound indicator reagent. The solid phase was contacted to an enzyme substrate signal producing component such that the enzyme component of the complexed indicator reagent would react with the substrate to produce a detectable signal. Prior to detection, the solid phase may undergo a third washing to remove unbound substrate. The signal which was detected was directly related to the amount of allergen-specific IgE antibodies in the test sample.

In one EIA procedure, the enzyme label was alkaline phosphatase, the substrate was 5-bromo-4-chloro-3-indolylphosphate and the detection or measurement step was performed with a reflectance spectrophotometer. The disc turned dark blue upon the addition of substrate to the solid phase, i.e., a positive assay result, when the serum sample contained IgE antibody specific to *Alternaria alternata*. The assay procedure was repeated using serum from different patients, and the results were found to correlate with the results obtained for the same serum samples using alternate tests, such as a radio-allergo-sorbent test (RAST) or a skin prick test as are well-known in the art.

### Example 2

In this experiment, the nitrocellulose disc used as the solid phase was one of many discs on a laminate composed of a mylar sheet to which a sheet of nitrocellulose had been glued. A circular shape was embossed onto the nitrocellulose sheet to form each of the discs. The micropores in the nitrocellulose sheet had diameters of about 450 nanometers. Each individual disc had a separate allergen attached thereto. Thus, the device could be used to detect the presence of antibodies to multiple allergens.

### Example 3

The procedure of Example 1 was repeated using 100 microliter portions of solutions containing birch allergen (137 µg) or dog allergen (280 µg) which were mixed with a 37% aqueous formaldehyde solution (12.5 µL) and incubated thereby forming pretreated allergen compositions. The amount of formaldehyde effective for pretreatment was found to range from about 10 µL to about 15 µL when the 37% aqueous formaldehyde solution was used. The compositions were used substantially in accordance with the procedures described in Examples 1 and 2 to produce devices which were then used to test serum samples. The assay results were found to correlate with the results of testing the same serum samples by other means: the disc turned dark blue when the serum sample contained IgE antibodies specific for the allergen immobilized upon the solid phase.

### Example 4

Tetrahydrofuran (25 µL) was mixed with 100 microliters of a solution containing Bermuda grass allergen (510 µg) in deionized water. The amount of tetrahydrofuran effective for pretreatment was found to range from about 10 µL to about 50 µL. The resulting mixture was incubated at 4°C for about 10 hours, and the incubated composition was allowed to stand at about 20°C for 30 to 60 minutes. The solution was then centrifuged, and the resultant supernatant, a pretreated Bermuda grass allergen composition, was decanted.

The procedure was repeated using 100 microliter-portions of solutions which contained Japanese cedar allergen (150 µg), June/Kentucky blue grass allergen (545 µg), perennial rye allergen (433 µg) or timothy allergen (43 µg) in deionized water. The pretreated allergen compositions were used to produce solid phase discs and were used in immunoassays substantially in accordance with the procedure described in Example 1. The assay results were found to correlate with the results of testing the same serum samples by other means: the disc turned dark blue when the serum sample contained IgE antibodies specific for the allergen immobilized upon the solid phase.

### Example 5

A 37 percent aqueous formaldehyde solution (15.6 µg) was mixed with 100 microliters of a solution containing mountain cedar allergen (733 µg) in deionized water. The resulting mixture was incubated at about 20°C for approximately 30 minutes. Tetrahydrofuran (28.7 µL) was then mixed with the incubated solution. The amount of formaldehyde effective for pretreatment was found to range from about 10 µL to about 20 µL , and the amount of tetrahydrofuran was found to range from about 10 µL to about 50 µL. The mixture was incubated for about 10 hours at 4°C and was allowed to stand at about 20°C for 30 to 60 minutes. The mixture was then centrifuged, and the resultant supernatant, a pretreated cedar allergen composition, was decanted.

This allergen pretreatment procedure was repeated, using 100 microliter-portions of solutions containing oak allergen (729 µg) or olive allergen (1670 µg), in deionized water, in place of the mountain cedar allergen. The pretreated allergen compositions were then used to produce immunoassay devices substantially in accordance with the procedures described in Examples 1 and 2. The EIA results were found to correlate with the results of testing of the same serum samples by other means: the disc turned dark blue when the serum sample contained IgE antibodies specific for the allergen immobilized upon the solid phase.

### Example 6

Aqueous NaCl (5 M, 12 µL) was mixed with 100 microliters of a solution containing *Cladosporium* (960 µg) in deionized water. The resulting mixture was incubated at about 4°C for about 10 hours, and the incubated composition was then allowed to stand at about 20°C for 30 to 60 minutes. The mixture was then centrifuged, and the resultant supernatant, a pretreated *Cladosporium* allergen composition, was decanted. Depending upon the molar value of the concentrated salt solution used, which value ranged from about 0.5 M to about 10 M, the amount of aqueous NaCl effective for pretreatment ranged from about 10 µL to about 20 µL.

The procedure was repeated using 100 microliters of a solution containing feather allergen (7 µg) in deionized water. The pretreated allergen compositions were then used to produce assay devices and were used in immunoassays substantially in accordance with the protocol described in Example 1. The assay results using the compositions and devices of the present invention were found to correlate with the results of testing the same serum samples by other means: the disc turned dark blue when the serum contacted thereto contained IgE antibodies specific for the allergen immobilized upon the solid phase.

### Example 7

An aqueous solution of 1-ethyl-3-(3-dimethylaminopropyl)carbodiimide (EDAC, 10 µL at 50 mg/mL) was mixed with 100 microliters of a solution containing *D*. *farinae* (280 µg) in deionized water. The amount of EDAC effective for the first stage of pretreatment ranged from about 5.0 µL to about 15 µL. The resulting mixture was incubated at about 22°C for about 15 minutes. A two microliter portion of a solution containing sodium borohydride (20 mg/mL, NaBH₄ ) in 10 µM phosphate buffered saline (pH 7) was mixed with the incubated solution, and the mixture was further incubated at about 4°C for 10 hours. The amount of NaBH₄ effective for the second stage of pretreatment ranged from about 1.0 µL to about 5.0 µL. The mixture was then allowed to stand at about 20°C for approximately 30 to 60 minutes. The mixture was centrifuged, and the resultant supernatant, a pretreated *D. farinae* allergen composition, was decanted.

The procedure was repeated using 100 microliters of a solution containing *D. pteronyssinus* (263 µg) in deionized water. The pretreated allergen compositions were used substantially in accordance with the procedures described in Example 1 to produce treated discs for immunoassays. The EIA results were found to correlate with the results of testing the same serum samples by other means: the disc turned dark blue when the serum sample contacted thereto contained IgE antibodies specific for the allergen immobilized upon the solid phase.

### Example 8

One hundred percent acetic acid (12.5 µL, with effective amounts ranging from about 5.0 µL to about 30 µL) was mixed with 100 microliters of a solution containing lamb's quarters allergen (1176 µg) in deionized water. The resultant mixture was incubated at about 22°C for approximately five minutes, after which time 6 N aqueous NaOH was added to adjust the pH to 7. The neutralized solution was incubated at about 4°C for 10 hours, and was then allowed to stand at about 20°C for 30 to 60 minutes. The mixture was then centrifuged, and the resultant supernatant, a pretreated lamb's quarters allergen composition, was decanted.

The procedure was repeated using 100 microliters of a solution containing mulberry allergen (40 µg) in deionized water. The pretreated allergen compositions were used to produce treated discs for enzyme immunoassays substantially in accordance with the procedures described in Example 1. The assay results were found to correlate with the results of testing the same serum samples by other means: the disc turned dark blue when the serum sample contacted thereto contained IgE antibodies specific for the allergen immobilized upon the solid phase.

### Example 9

A solution of 6 N aqueous HCl (24 µL, with effective amounts ranging from about 6.0 µL to about 30 µL) was mixed with 100 microliters of a solution containing *Penicillium* (120 µg). The resulting mixture was incubated for approximately five minutes at about 20°C, after which time 6 N aqueous NaOH was added to adjust the pH to 7. The neutralized solution was incubated at 4°C for 10 hours and was then allowed to stand at about 20°C for 30 to 60 minutes. The mixture was then centrifuged, and the resultant supernatant, a pretreated *Penicillium* allergen composition, was decanted.

The allergen pretreatment procedure was repeated with 100 microliters of a solution containing *Parietaria* allergen (400 µg) in deionized water. The solutions were then used substantially in accordance with the procedures described in Example 1 to produce discs and to test serum samples in an EIA. The assay results were found to correlate with the results of testing the same serum samples by other means: the disc turned dark blue when the serum contacted thereto contained IgE antibodies specific for the allergen immobilized upon the solid phase.

### Example 10

Untreated allergen compositions included from about 0.5 to about 50 *Aspergillus* allergen; from about 0.6 to about 20.6 of cat allergen; from about 0.1 to about 10.0 of elm allergen; from about 0.4 to about 100 of house dust allergen; from about 0.1 to about 11.5 of maple allergen; from about 0.3 to about 90.4 of mugwort allergen and from about 1.7 to about 130.4 of plantain allergen in deionized water.

The solutions were then used substantially in accordance with the procedures described in Example 1 to produce discs and to test serum samples in an EIA. The assay results were found to correlate with the results of testing the same serum samples by other means: the disc turned dark blue when the serum sample contained IgE antibodies specific for the allergen immobilized upon the solid phase.

### Example 11

Pretreated allergen compositions, which were produced as described in Examples 1, and 3 through 9, and which differed from one another with respect to allergen content, were used to test for IgE antibodies in a series of serum samples. Upper and lower allergen concentration limits were set by classifying a pretreated allergen composition as either "too dilute" if that composition failed to produce a maximum positive IgE antibodies test result with a serum sample which had tested positive with a more concentrated allergen solution, or "too concentrated" if the composition failed to produce a maximum positive IgE antibodies test result with a serum sample which had tested positive with a less concentrated allergen solution. The allergen concentrations tested ranged from about 0.05 milligrams of allergen per milliliter of water, prior to pretreatment, to about 170 milligrams/milliliter. The test results are presented in Table 1 and illustrate the most effective concentration ranges for each of the allergens tested.

**Table 1**

| Allergen | Effective concentration range (protein content in solution) |
|---|---|
| *Alternaria alternata* allergen | from 0.05 to 4.0 mg/mL |
| *Aspergillus fumigatus* allergen | from 0.5 to 50.0 mg/mL |
| Bermuda grass allergen | from 0.8 to 81.6 mg/mL |
| birch allergen | from 0.1 to 6.0 mg/mL |
| mountain cedar allergen | from 0.04 to 4.5 mg/mL |
| Japanese cedar allergen | from 0.1 to 20.5 mg/mL |
| *Cladosporium* allergen | from 0.05 to 38.4 mg/mL |
| cat allergen | from 0.6 to 20.6 mg/mL |
| dog allergen | from 1.3 to 38.4 mg/mL |
| *D. farinae* allergen | from 0.7 to 22.4 mg/mL |
| *D. pteronyssinus* allergen | from 0.6 to 84.2 mg/mL |
| elm allergen | from 0.1 to 146.0 mg/mL |
| feather allergen | from 0.02 to 0.2 mg/mL |
| giant ragweed allergen | from 0.2 to 148.2 mg/mL |
| house dust allergen | from 0.4 to 100 mg/mL |
| June/Kentucky bluegrass allergen | from 0.05 to 21.8 mg/mL |
| lamb's quarters allergen | from 0.2 to 47.0 mg/mL |
| maple allergen | from 0.1 to 166.3 mg/mL |
| mugwort allergen | from 0.3 to 90.4 mg/mL |
| mulberry allergen | from 0.1 to 12 mg/mL |
| oak allergen | from 0.2 to 29.2 mg/mL |
| olive allergen | from 0.1 to 66.8 mg/mL |
| *Parietaria* allergen | from 1.0 to 40.0 mg/mL |
| plantain allergen | from 1.7 to 130.4 mg/mL |
| *Penicillium* allergen | from 0.1 to 4.8 mg/mL |
| perennial rye allergen | from 0.05 to 17.3 mg/mL |
| short ragweed allergen | from 0.2 to 151.6 mg/mL |
| timothy allergen | from 0.05 to 6.6 mg/mL |

In this manner, the optimum concentration of allergen was determined for the production of solid phase assay devices.

### Example 12

A milk protein composition comprising milk protein extract, casein and β-lactoglobulin was prepared as follows. Casein powder (Greer Laboratories, Lenoir, North Carolina) was dissolved in de-ionized water at approximately 4 mg/ml. β-lactoglobulin powder (Sigma Chemical Company, St. Louis, Missouri) was dissolved in de-ionized water at approximately 72 mg/ml. The casein solution and the β-lactoglobulin solution were combined in a 1:1 volume ratio. Bovine milk protein extract powder ("Milk, Cow" from Greer Laboratories, Lenoir, North Carolina) was dissolved in de-ionized water at approximately 1.4 mg/ml. The β-lactoglobulin-casein solution was combined with the milk solution in a 1:1 volume ratio. The β-lactoglobulin-casein-milk solution was acidified by the addition of 6N HCl to make a final concentration of 0.06N HCl. After 5 minutes, the solution was neutralized by the addition of 6N NaOH followed by 1 M HEPPS buffer, pH 8.3, to make a final concentration of 0.1M HEPPS buffer. This solution was stored at 2-8°C for approximately 3 days. Before use, the solution was centrifuged to provide a clear solution which may be adsorbed onto nitrocellulose.

### Example 13

A milk protein composition comprising milk protein extract, casein, β-lactoglobulin and BGG was prepared as follows. Casein powder (Greer Laboratories, Lenoir, North Carolina) was dissolved in de-ionized water at approximately 8 mg/ml. β-lactoglobulin powder (Sigma Chemical Company, St. Louis, Missouri) was dissolved in de-ionized water at approximately 144 mg/ml. The casein solution and the β-lactoglobulin solution were combined in a 1:1 volume ratio. Bovine milk protein extract powder ("Milk, Cow" from Greer Laboratories, Lenoir, North Carolina) was dissolved in de-ionized water at approximately 2.8 mg/ml. The β-lactoglobulin-casein solution was combined with the milk solution in a 1:1 volume ratio. The β-lactoglobulin-casein-milk solution was acidified by the addition of 6N HCl to make a final concentration of 0.06N HCl. After 5 minutes, the solution was neutralized by the addition of 6N NaOH followed by 1M HEPPS buffer, pH 8.3, to make a final concentration of 0.2M HEPPS buffer. Bovine gamma globulin (BGG) powder (Sigma Chemical Company, St. Louis, Missouri) was dissolved in de-ionized water at approximately 10 mg/ml. The β-lactoglobulin-casein-milk solution was then combined with the BGG solution in a 1:1 volume ratio. This solution was stored at 2-8°C for approximately 3 days. Before use, the solution was centrifuged to provide a clear solution which may be adsorbed onto nitrocellulose.

### Example 14

First test cards 82 with a test site 84 (see Figures 1 and 2) containing a milk protein composition of only milk protein extract powder ("Milk, Cow" from Greer Laboratories, Lenoir, North Carolina) were prepared by depositing 2 microliters of approximately a 0.7 mg/ml solution (0.1M HEPPS buffer in de-ionized water), pretreated with acid according to the method in Examples 12 and 13, onto the test site and drying the test site at room temperature overnight.

Second test cards 82 with a test site 84 containing a milk protein composition of milk protein extract, casein and β-lactoglobulin were prepared by depositing 2 microliters of the milk protein composition solution prepared according to Example 12 onto a test site and drying the test site at room temperature overnight.

Third test cards 82 with a test site 84 containing a milk protein composition of milk protein extract, casein, β-lactoglobulin and BGG were prepared by depositing 2 microliters of the milk protein composition solution prepared according to Example 13 onto a test site and drying the test site at room temperature overnight.

Pools of human plasma containing high levels of anti-milk-protein IgE antibodies were prepared as follows. Human plasma samples were tested for anti-milk-protein IgE antibodies using Phadebas RAST® (Pharmacia) Milk test. Samples were combined to produce plasma pools having at least 1.1 PRU/ml (expressed in Pharmacia Radioimmunoassay Units (PRU) per milliliter) of anti-milk-protein IgE antibodies. The plasma pools were then serially diluted with about 0.1M TRIS buffer saline (pH 7.2) containing serum proteins to produce pooled plasma samples containing about 0.1, 0.17, 0.22, 0.34 and 1.12 PRU/ml of anti-milk-protein IgE antibodies.

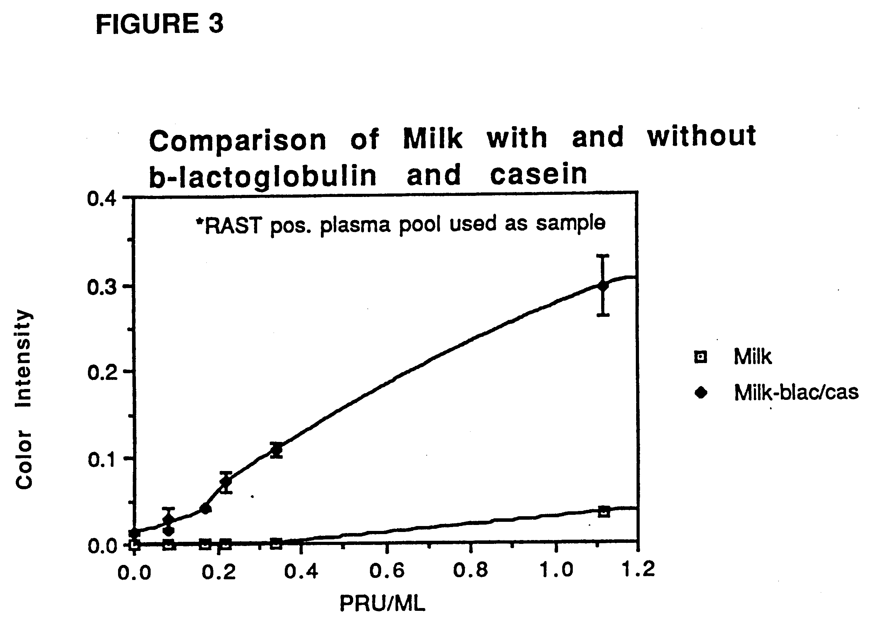

Using an Abbott Matrix® Analyzer (commercially available from Abbott Laboratories, North Chicago, Illinois), the anti-milk-protein IgE antibody positive plasma pools were analyzed for the presence of detectable amounts of anti-milk-protein IgE antibodies using the first (milk protein extract only) and second (milk protein extract, casein and β-lactoglobulin) test cards prepared above. The presence of IgE antibodies from the sample pools captured on the solid phase was detected by incubating the solid phase with alkaline phosphatase labeled goat anti-human-IgE IgG antibodies, washing the solid phase with buffer and adding the enzyme substrate 5-bromo-4-chloro-1-indolylphosphate (BCIP). The intensity of the color developed on the solid phase was measured with a reflectance detector. After subtracting background intensity, the relative color intensity of the samples analyzed on each test card were plotted against the concentration of IgE antibodies as measured by Phadebas RAST® (PRU/ml). The plot, as shown in Figure 3, illustrates the significant improvement in sensitivity realized by the use of the milk protein composition of Example 12 on the second test cards above.

Using an Abbott Matrix® Analyzer, nine (9) human plasma (anti-milk-protein IgE positive by Phadebas RAST® Milk test) patient samples were analyzed for the presence of detectable amounts of anti-milk-protein IgE antibodies using the second and third test cards prepared above. The second test cards (without BGG) detected anti-milk-protein IgE antibodies in eight (8) of the nine (9) samples tested, whereas the third test cards (with BGG) detected anti-milk-protein IgE antibodies in nine (9) of the nine (9) samples tested. As described above, the presence of IgE antibodies from the sample captured on the solid phase was detected by incubating the solid phase with alkaline phosphatase labeled goat anti-human-IgE IgG followed by the addition of the enzyme substrate BCIP. The color intensity was again measured by a reflectance detector and adjusted for the background intensity.

### Example 15

A wheat protein composition comprising wheat allergen extract was prepared as follows. Wheat Flour (Greer Laboratories, Lenoir, North Carolina) was dissolved in de-ionized water at approximately 5 mg/ml. A solution of 1M HEPPS at pH 8.3 was added to the wheat flour solution to make a final concentration of 0.2M HEPPS. The allergen solution was heated in a water bath at 80°C for 3 minutes with agitation, then the solution was placed in ice water for 3 minutes with shaking. This solution was centrifuged to provide a clear solution which may be adsorbed onto nitrocellulose.

### Example 16

First test cards 82 with a test site 84 (see Figures 1 and 2) containing a wheat protein composition of only wheat allergen extract powder (Wheat Flour from Greer Laboratories) were prepared by depositing 2 microliters of approximately a 5 mg/ml solution (in de-ionized water) onto the test site and drying the test site at room temperature overnight.

Second test cards 82 with a test site 84 containing a wheat protein composition of wheat allergen extract powder in HEPPS buffer were prepared by depositing 2 microliters of the wheat protein composition prepared according to Example 15 onto a test site and drying the test site at room temperature overnight.

Pools of human plasma containing high levels of anti-wheat protein IgE antibodies were prepared as follows. Human plasma samples were tested for anti-wheat protein IgE antibodies using Phadebas RAST® Wheat test (Pharmacia). Samples were combined to produce plasma pools having at least 6.3 PRU/ml (expressed in Pharmacia Radioimmunoassay Units (PRU) per milliliter) of anti-wheat protein IgE antibodies. The plasma pools were then serially diluted with about 0.1M TRIS buffer saline (pH 7.2) containing serum proteins to produce pooled plasma samples containing about 1.10, 3.62, and 6.31 PRU/ml of anti-wheat protein IgE antibodies.

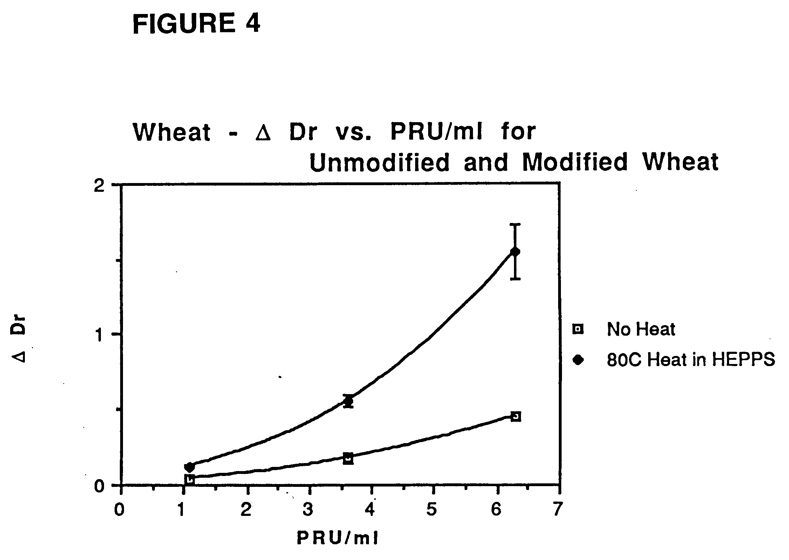

Using an Abbott Matrix® Analyzer (commercially available form Abbott Laboratories, North Chicago, Illinois), the anti-wheat protein IgE antibody positive plasma pools were analyzed for the presence of detectable amounts of anti-wheat protein IgE antibodies using the first (wheat, unheated) and second (wheat, heated) test cards prepared above. The presence of IgE antibodies from the sample pools captured on the solid phase was detected by incubating the solid phase with alkaline phosphatase labeled anti-human-IgE IgG antibodies, washing the solid phase with buffer and adding the enzyme substrate 5-bromo-4-chloro-1-indolylphosphate (BCIP). The intensity of the color developed on the solid phase was measured with a reflectance detector. After subtracting background intensity, the relative color intensity of the samples analyzed on each test card were plotted against the concentration of IgE antibodies as measured by Phadebas RAST® (PRU/ml). The plot, as shown in Figure 4, illustrates the significant improvement in sensitivity realized by the use of the wheat protein composition of Example 15 on the second test cards above.

Using an Abbott Matrix® Analyzer, 44 human plasma (anti-wheat protein IgE positive by Phadebas RAST® Wheat test) patient samples were analyzed for the presence of detectable amounts of anti-wheat protein IgE antibodies using the first and second test cards prepared above. The first test cards containing wheat not treated with heat detected anti-wheat protein IgE antibodies in 31 of the 44 samples tested, whereas the second test cards containing wheat treated with heat in HEPPS buffer detected anti-wheat protein IgE antibodies in 37 of the 44 samples tested. As described above, the presence of IgE antibodies from the sample captured on the solid phase was detected by incubating the solid phase with alkaline phosphatase labeled goat anti-human-IgE IgG followed by the addition of the enzyme substrate BCIP. The color intensity was again measured by a reflectance detector and adjusted for the background intensity.

It will be appreciated by one skilled-in-the-art that the concepts of the present invention are equally applicable to many different allergens (specific binding members), solid phase materials and immunoassay protocols. It will also be appreciated that the selection of any given label, ancillary binding member or solid phase material is generally not critical to the present invention. The materials are selected to optimize the results provided by the chosen assay configuration. The embodiments described herein are intended as examples rather than as limitations. Thus, the description of the invention is not intended to limit the invention to the particular embodiments described in detail, but it is intended to encompass all equivalents and subject matter within the spirit and scope of the invention as described above and as set forth in the following claims.

## Claims

1. A method of detecting anti-wheat-protein antibodies in a sample suspected of having said antibodies comprising:
contacting the sample with a solid phase having immobilized thereon a wheat allergen extract; and
detecting the presence or amount of antibodies bound to said solid phase.

2. The method of claim 1 wherein the antibodies are IgE antibodies.

3. The method of claim 1 wherein the solid phase is selected from the group consisting of cellulose, cellulose acetate, nitrocellulose, acetate/nitrate mixed ester cellulose, silica, fiberglass, and agarose.

4. The method of claim 1 wherein said wheat allergen extract is pretreated with heat prior to immobilization on the solid phase.

5. The method of claim 2 wherein the IgE antibodies are detected by contacting the solid phase with an indicator reagent specific for IgE antibodies and measuring the amount of indicator reagent bound to the solid phase.

6. The method of claim 5 wherein said indicator reagent comprises a label conjugated to a binding member specific for a member selected from the group consisting of allergen, IgE and an ancillary specific binding member.

7. The method of claim 6, wherein said indicator reagent comprises a label conjugated to an anti-IgE antibody or anti-IgE antibody fragment.

8. The method of claim 7, wherein said label is a member selected from the group consisting of chromogens, catalysts, fluorescent compounds, chemiluminescent compounds, radioactive isotopes, colloidal metallic particles, colloidal selenium particles, dye particles, enzymes, organic polymer latex particles and liposomes or other vesicles containing signal producing components.

9. The method of claim 5, further comprising the step of washing unbound indicator reagent from said solid phase prior to detecting the presence or amount of IgE in the test sample.

10. The method of claim 1, wherein said solid phase is a planar sheet.

11. The method of claim 4, wherein said wheat allergen extract is pretreated by heating said wheat allergen extract in an aqueous buffer at a pH within the range of about 7.0 to about 9.0.

12. The method of claim 11, wherein said buffer is HEPPS buffer at a concentration within the range of about 0.01M to about 10M.

13. The method of claim 12, wherein said wheat allergen extract is heated to a temperature within the range of about 50°C to about 100°C for about 1 minute to about 20 minutes.

14. The method of claim 4, wherein said wheat allergen extract is pretreated by heating said wheat allergen extract in 0.2M HEPPS buffer at pH 8.3 at 80°C for three minutes.

15. A composition comprising heat treated wheat allergen extract.

16. The composition of claim 15 wherein said heat treatment comprises heating said wheat allergen extract in an aqueous buffer at a pH within the range of about 7.0 to about 9.0.

17. The composition of claim 16 wherein said buffer is HEPPS buffer at a concentration within the range of about 0.01M to about 10M.

18. The composition of claim 16 wherein said wheat allergen extract is heated to a temperature within the range of about 50°C to about 100°C for about 1 minute to about 20 minutes.

19. The composition of claim 15 wherein said wheat allergen extract is pretreated by heating said wheat allergen extract in 0.2M HEPPS buffer at pH 8.3 at 80°C for three minutes.

20. A composition comprising nitrocellulose containing heat treated wheat allergen extract.

21. The composition of claim 20 wherein said heat treatment comprises heating said wheat allergen extract in an aqueous buffer at a pH within the range of about 7.0 to about 9.0.

22. The composition of claim 21 wherein said buffer is HEPPS buffer at a concentration within the range of about 0.01M to about 10M.

23. The composition of claim 21 wherein said wheat allergen extract is heated to a temperature within the range of about 50°C to about 100°C for about 1 minute to about 20 minutes.

24. The composition of claim 20 wherein said wheat allergen extract is pretreated by heating said wheat allergen extract in 0.2M HEPPS buffer at pH 8.3 at 80°C for three minutes.
